(19) **Europäisches Patentamt**

**European Patent Office**

**Office européen des brevets**

(11) Veröffentlichungsnummer: **0 437 254 B1**

(12) **EUROPÄISCHE PATENTSCHRIFT**

(45) Veröffentlichungstag der Patentschrift: **27.12.95**

(51) Int. Cl.[6]: **C12Q 1/34**, C12Q 1/50

(21) Anmeldenummer: **91100241.8**

(22) Anmeldetag: **09.01.91**

(54) **Verfahren und Reagenz zur enzymatischen Bestimmung von Creatinin**

(30) Priorität: **11.01.90 DE 4000668**

(43) Veröffentlichungstag der Anmeldung:
**17.07.91 Patentblatt 91/29**

(45) Bekanntmachung des Hinweises auf die
Patenterteilung:
**27.12.95 Patentblatt 95/52**

(84) Benannte Vertragsstaaten:
**AT BE CH DE DK ES FR GB GR IT LI LU NL SE**

(56) Entgegenhaltungen:

ANALYTICAL LETTERS, Band 21, Nr. 6, 1988,
New York, NY (US); J. SIEDEL et al., Seiten
1009-1017/

CLINICAL CHEMISTRY, Band 28, Nr. 7, 1982,
Winston-Salem, NC (US); E. TANGANELLI et
al., Seiten 1461-1464/

(73) Patentinhaber: **BOEHRINGER MANNHEIM GMBH**

**D-68298 Mannheim (DE)**

(72) Erfinder: **Siedel, Joachim, Dr.**
**Weilheimer Strasse 10**
**W-8139 Bernried (DE)**
Erfinder: **Vogt, Bernd, Dr.**
**Herrestrasse 14**
**W-8132 Tutzing (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

**Beschreibung**

Die Erfindung betrifft ein Verfahren und Reagenz zur enzymatischen Bestimmung von Creatinin in wäßrigen Proben, insbesondere biologischen Ursprungs wie Serum und Urin.

Die Bestimmung der Creatinin-Konzentration im Serum und Urin stellt in der klinischen Chemie eine der wichtigsten Methoden zur Diagnostik der Nierenfunktion dar. Gegenüber der für diesen Zweck ebenfalls häufig eingesetzten Harnstoffbestimmung hat sie den entscheidenden Vorteil, daß die Konzentration des Creatinins im Serum und Urin von der Ernährungsweise, insbesondere von der Zufuhr proteinreicher Nahrung, praktisch unbeeinflußt bleibt.

Allerdings ist im Vergleich zum Harnstoff die Konzentration des Creatinins z.B. im Serum im Entscheidungsbereich (Obergrenze der Normalwerte 1,10 mg/dl bei Männern bzw. 0,90 mg/dl bei Frauen) außerordentlich gering. Deshalb müssen an die Empfindlichkeit und Spezifität eines geeigneten Creatinin-Tests hohe Anforderungen gestellt werden.

Wegen seiner Bedeutung als Standarduntersuchungsmethode im klinischen Labor sollte er aber gleichzeitig auch mit möglichst geringem Arbeitsaufwand durchführbar und besonders für den Einsatz an Analysenautomaten geeignet sein.

Die auch heute noch gebräuchlichste Bestimmungsmethode für Creatinin beruht auf dem Verfahren nach Jaffé, bei dem die Probe mit alkalischer Pikrinsäure-Lösung vermischt und der sich durch die Reaktion von Creatinin mit Pikrat bildende rote Farbstoff gemessen wird.

Dieses an sich einfache Verfahren ist jedoch mit einer Reihe wesentlicher Nachteile behaftet. Neben der Notwendigkeit der Verwendung teilweise ätzender und giftiger Reagenzien sind dies insbesondere positive oder negative Störungen durch sogenannte "Pseudo-Creatinin-Chromogene". Mehr als 50 solcher Chromogene, darunter natürlicherweise im Serum oder Urin vorkommende Bestandteile wie Glucose, Pyruvat und Acetoacetat, sind in der Literatur beschrieben (vgl. Clin. Chem. 1980: 26, 1119 - 1126). Eine Vielzahl von Modifikationen der Jaffé-Methode zur Vermeidung dieser Störungen wurden entwickelt, ohne sie damit jedoch vollständig beheben zu können.

Um die Spezifität der Creatinin-Bestimmung zu verbessern, wandte sich in den vergangenen Jahren die Aufmerksamkeit mehr und mehr enzymatischen Testverfahren zu.

So ist in Scand. J. Clin. Lab. Invest., suppl. 29 p. 126 (1972) ein vollenzymatischer Creatinintest beschrieben, bei dem Creatinin mittels der Creatinin-Amidohydrolase (EC 3.5.2.10) in Creatin umgewandelt, letzteres mit ATP und Creatin-Kinase (EC 2.7.3.2) zu Creatinphosphat umgesetzt und das enstehende ADP in einer gekoppelten Reaktion mit Pyruvat-Kinase (EC 2.7.1.40) und Lactat-Dehydrogenase (EC 1.1.1.27) photometrisch im UV-Bereich über den NADH-Verbrauch in der Reaktionslösung gemessen wird.

Diese Methode ist zwar spezifisch für Creatinin, sie weist jedoch wegen des Verbrauchs von nur einem Molekül NADH pro Molekül Creatinin, selbst bei Einsatz größerer Probevolumina im Testansatz (200 $\mu$l Probe/1000 $\mu$l Reagenz), eine aufgrund des vergleichsweise niedrigen molaren Extinktionskoeffizienten von NADH relativ geringe Empfindlichkeit und damit auch Präzision im Entscheidungsbereich auf.

Außerdem kann es bei erhöhten Konzentrationen an Creatin und/oder Pyruvat in der Probe zu einem unspezifischen Überverbrauch an NADH und damit ggfs. zu falsch niedrigen Creatinin-Wiederfindungen kommen.

Schließlich ist auch die Applizierbarkeit an Analysenautomaten wegen des relativ langsamen Reaktionsablaufs stark eingeschränkt.

Ein anderes, ebenfalls für Creatinin spezifisches Testprinzip beruht auf dem Abbau von Creatinin zu 1-Methylhydantoin und $NH_4^+$ mittels der Creatinin-Iminohydrolase (E.C. 3.5.4.21); das entstehende $NH_4^+$ wird über die NADH-Abnahme in einer gekoppelten Glutamat-Dehydrogenase-Reaktion photometrisch im UV-Bereich gemessen (Clin. Chem. 1982: 28, 1461 - 1464).

Wegen des vergleichsweise raschen Reaktionsablaufs ist dieses Verfahren zwar prinzipiell gut für die Verwendung an automatisierten Analysengeräten geeignet, bezüglich seiner Meßempfindlichkeit und Präzision gelten jedoch hier ebenfalls wegen des Verbrauchs von nur einem Molekül NADH pro Molekül Creatinin die gleichen Einschränkungen wie für den zuerstgenannten UV-Test.

Die Hauptnachteile der vorgenannten enzymatischen Creatinintests, d.h. zu geringe Nachweisempfindlichkeit und damit zu hohe Impräzision im Entscheidungsbereich, lassen sich vermeiden, wenn man Creatinin enzymatisch zu Sarcosin abbaut, entweder mittels der Enzyme Creatinin-Amidohydrolase (E.C. 3.5.2.10) und Creatin-Amidinohydrolase (E.C. 3.5.3.3)(Clin. Chem. 1984: 30, 968) oder mittels der Enzyme Creatinin-Iminohydrolase, ATP-abhängiger 1-Methylhydantoinase und N-Carbamoylsarcosin-Amidohydrolase (Anal. Letters 1988: 21, 1009 - 1017). Das entstehende Sarcosin wird in Gegenwart der Sarcosin-Oxidase (E.C. 1.5.3.1) unter anderem zu $H_2O_2$ umgesetzt, das sich durch oxidative Kupplung von z.B. 2,4,6-Tribrom-3-hydroxybenzoesäure mit entweder 4-Aminoantipyrin (4-AAP) oder 3-Methyl-2-benzo-(2'-sulfo)-thiazolinon-hydrazon (MBTH-S) in Gegenwart von Peroxidase (E.C. 1.11.1.7) auf empfindliche Weise colorimetrisch messen läßt.

Diese Indikatorsysteme sind jedoch störanfällig gegenüber reduzierenden Probenbestandteilen wie insbesondere Ascorbinsäure, Bilirubin sowie bestimmten Pharmaka (Ca-Dobesilat, $\alpha$-Methyldopa), die mit der Farbbildung negativ interferieren.

Dagegen sind auf NADH- bzw. NADPH-abhängigen Dehydrogenase-Indikatorreaktionen basierende Meßverfahren gegenüber solchen Substanzen praktisch inert.

Es besteht daher der Bedarf an einem Verfahren zur spezifischen enzymatischen Bestimmung von Creatinin, das die Vorteile von Tests mit auf $H_2O_2$-Visualisierung basierenden Indikatorsystemen, d.h. gute Meßempfindlichkeit und Präzision auch bei niedrigen Analyt-Konzentrationen in der Probe, mit denen auf NAD(P)H-abhängigen Dehydrogenase-Reaktionen beruhender Nachweismethoden, d.h. Störunanfälligkeit gegenüber unspezifisch reduzierenden Probenbestandteilen wie insbesondere Ascorbinsäure, Bilirubin, Ca-Dobesilat und $\alpha$-Methyldopa, miteinander verbindet und gleichzeitig auch eine gute Applizierbarkeit an automatisierten Analysengeräten erlaubt.

Das Verfahren sollte darüberhinaus auch negative Einflüsse durch endogenes Creatin und Pyruvat aus der Probe auf die Creatinin-Wiederfindung eliminieren.

Diese Aufgabe wird erfindungsgemäß gelöst durch ein Verfahren zur enzymatischen Bestimmung von Creatinin in wäßrigen Lösungen, welches dadurch gekennzeichnet ist, daß man das in der Probe enthaltene Creatinin mittels der Creatinin-Iminohydrolase zu 1-Methylhydantoin und einem ersten Molekül $NH_3$ umsetzt, das gebildete 1-Methylhydantoin mittels der ATP-abhängigen 1-Methylhydantoinase und der N-Carbamoylsarcosin-Amidohydrolase zu Sarcosin, $CO_2$ und einem zweiten Molekül $NH_3$ umsetzt und beide gebildeten Moleküle $NH_3$ gemeinsam bestimmt. Diese Bestimmung erfolgt vorzugsweise durch Umsetzung von $NH_3$ mit $\alpha$-Ketoglutarat in Gegenwart von Glutamat-Dehydrogenase und NADH bzw. NADPH, wobei Glutamat gebildet und der Verbrauch an NADH bzw. NADPH, insbesondere photometrisch gemessen wird.

Das erfindungsgemäße Verfahren ermöglicht es, die Nachweisempfindlichkeit gegenüber der bekannten Creatinin-Bestimmungsmethode dieser Art bei gleichen Probe- zu Reagenz-Volu-Volumenverhältnissen zu verdoppeln, die Meßpräzision entsprechend zu verbessern und gleichzeitig die Nachteile von über $H_2O_2$ ablaufenden colorimetrischen Nachweisverfahren wie Störungen durch unspezifisch reduzierende Probenbestandteile zu vermeiden.

Außerdem haben endogenes Creatin bzw. Pyruvat aus der Probe keinen Einfluß auf die Creatinin-Wiederfindung, da die Nachweisreaktion nicht über diese Substanzen abläuft.

Da Probenmaterialien wie Serum und Urin stets Spuren von endogenen Ammoniumionen aufweisen, besteht eine besonders bevorzugte Ausführungsform des Verfahrens zur exakten Creatinin-Bestimmung darin, daß die zu analysierende Probe in einer ersten Stufe mit $\alpha$-Ketoglutarat, NAD(P)H, GIDH, NMHase sowie ggfs. CSHase zur Reaktion gebracht, nach komplettem Ablauf ein erster NAD(P)H-Verbrauch gemessen wird, anschließend in einer zweiten Stufe mit CRIMI, ATP sowie ggfs. CSHase zu einer weiteren Reaktion gebracht, ein zweiter NAD(P)H-Verbrauch gemessen und der Gehalt an Creatinin in der Probe aus dem NAD(P)H-Verbrauch in der zweiten Reaktionsstufe berechnet wird (Stufenmessung, E1/E2-Verfahren).

Wird in der zweiten Reaktionsstufe, je nach dem gewählten bzw. vom Analysengerät her verfügbaren Inkubationsintervall nicht der komplette Substratumsatz erreicht, so kann anstelle einer Endpunktmessung im sogenannten kinetischen 'fixed-time'-Modus gearbeitet werden. Um dabei eine lineare Beziehung zwischen NAD(P)H-Verbrauch im Test und der Creatinin-Konzentration in der Probe zu gewährleisten, muß die Reaktionskinetik nach erster bzw. pseudo-erster Ordnung ablaufen. Da bei dem erfindungsgemäßen Verfahren die beiden aus Creatinin herrührenden $NH_3$-Moleküle nacheinander aus zwei verschiedenen Stufen der Abbausequenz für die GIDH-Indikator-Reaktion abgegriffen werden, ist es in diesem Fall erforderlich, daß entweder die CRIMI- oder die GIDH-Reaktion den geschwindigkeitsbestimmenden Schritt im Testansatz darstellen.

In einer anderen Ausführungsweise des erfindungsgemäßen Verfahrens kann auch mit der sogenannten Probe-/Probenleerwert-Methode gearbeitet werden; für den Probenleerwert wird aus dem Gesamtreagenz die CRIMI weggelassen (Leerwertreagenz) und die Creatinin-Konzentration in der Probe aus der Differenz des NAD(P)H-Verbrauchs bei Inkubation von Probe mit Gesamtreagenz einerseits und von Probe mit Leerwertreagenz andererseits berechnet.

Ein weiterer Gegenstand der Erfindung ist ein Reagenz zur enzymatischen Bestimmung von Creatinin, dadurch gekennzeichnet, daß es in einer wäßrigen, gepufferten Lösung Creatinin-Iminohydrolase, N-Carbamoylsarcosin-Amidohydrolase, ATP-abhängige 1-Methylhydantoin-Hydrolase, ATP, $Mg^{++}$ und gegebenenfalls Glutamat-Dehydrogenase, NADH oder NADPH, $\alpha$-Ketoglutarat sowie ggfs. Enzymaktivatoren wie ADP und $K^+$-Salz enthält.

Die Bestimmung des während der Umsetzung von Creatinin zu Sarcosin gebildeten Ammoniaks geschieht bevorzugt, wie oben beschrieben, durch enzymatische Umsetzung von $\alpha$-Ketoglutarat zu Glutamat mit Hilfe von Glutamat-Dehydrogenase (EC 1.4.1.3), wobei NADH bzw. NADPH zu $NAD^+$ bzw. $NADP^+$ oxidiert werden.

Die Bestimmung des Ammoniaks kann jedoch prinzipiell auch auf andere Weise erfolgen, wobei jedoch bei der Wahl des Bestimmungsverfahrens darauf zu achten ist, daß die Anwesenheit von Proteinen den Ammoniak-Nachweis nicht stört. Auf solche Verfahren wird z.B. in Methoden der enzymatischen Analyse, Band II, 3. Auflage, 1974 , herausgegeben von H.U. Bergmeyer, Verlag Chemie Weinheim, hingewiesen.

Insbesondere ist auch ein $NH_3$-Nachweis mittels eines pH-Indikators wie z.B. Bromphenolblau möglich, wie in Clin.Chem. 29 (1983) 645-649 beschrieben. Dazu ist es erforderlich, daß die Reagenzbestandteile (ohne Glutamat-Dehydrogenase) in einer Reaktionsschicht aus saugfähigem Material (z.B. Gelatine) vorliegen, die durch eine semipermeable Membran von einer Indikatorschicht getrennt ist. Bei Zugabe des Serums zur Reaktionsschicht entsteht $NH_3$, der durch die semipermeable Membran diffundieren kann und mit dem Indikator reagiert. Anschließend kann aufgrund der Verfärbung des Indikators die $NH_3$-Menge und damit auch die Creatininmenge bestimmt werden (siehe Clin.Chem. 29 (1983) 645-649).

Der Gehalt an endogenem $NH_3$ kann - entsprechend dem Bestimmungsverfahren in flüssigem Medium - in einer Reaktionsschicht mit dem Gesamtreagenz ohne CRIMI (und auch ohne Glutamat-Dehydrogenase) bestimmt werden.

Der pH-Wert der gepufferten wäßrigen Lösung liegt zweckmäßig zwischen pH 7,0 und 9,0, bevorzugt zwischen pH 7,3 und 8,7, insbesondere zwischen pH 7,7 und 8,4. Zum Einstellen des pH-Wertes kommen Substanzen in Frage, die im genannten pH-Bereich eine ausreichende Pufferkapazität aufweisen, wie z.B. Phosphat, TRIS, Triethanolamin oder TES. Bevorzugt wird insbesondere TRIS-Puffer. Die Konzentration des Puffers liegt normalerweise zwischen 20 und 500 mmol/l, vorzugsweise zwischen 50 und 200 mmol/l, ganz besonders bevorzugt zwischen 75 und 150 mmol/l.

Die Creatinin-Iminohydrolase wird zweckmäßig mit 0.2 bis 20 U/ml, vorzugsweise 0,5 bis 15 U/ml, insbesondere 1 bis 10 U/ml eingesetzt.

Die 1-Methylhydantoinase wird normalerweise zwischen 0,1 und 10 U/ml, vorzugsweise zwischen 0,2 und 5 U/ml, insbesondere zwischen 0,4 und 2 U/ml eingesetzt.

Für die katalytische Konzentration der N-Carbamoylsarcosin-Amidohydrolase haben sich Werte zwischen 0,5 bis 20 U/ml, insbesondere zwischen 1 bis 15 U/ml, ganz bevorzugt zwischen 2 bis 10 U/ml, als günstig erwiesen.

Im Falle der Glutamat-Dehydrogenase werden normalerweise pro ml Reaktionsgemisch 10 bis 200 U, vorzugsweise 20 - 150 U, insbesondere 40 bis 125 U eingesetzt.

Die Konzentration an NADH - oder alternativ NADPH - beträgt 150 bis 400 $\mu$mol/l, vorzugsweise 200 bis 350 $\mu$mol/l.

$\alpha$-Ketoglutarat wird zweckmäßig in Konzentrationen zwischen 1 und 30 mmol/l, vorzugsweise zwischen 5 und 20 mmol/l, insbesondere zwischen 8 bis 17 mmol/l eingesetzt.

Die Konzentration an ATP beträgt zweckmäßigerweise 0,05 bis 50 mmol/l, vorzugsweise 0,5 bis 20 mmol/l, besonders bevorzugt 1 bis 15 mmol/l.

$Mg^{++}$ wird in Form wasserlöslicher Salze wie insbesondere $MgCl_2$ oder Mg-Aspartat eingesetzt, in Konzentrationen zwischen 0,1 und 50 mmol/l, vorzugsweise zwischen 0,5 und 20 mmol/l, insbesondere zwischen 1 und 10 mmol/l.

Zur Aktivitätssteigerung der 1-Methylhydantoinase haben sich Zusätze von K-Salzen wie insbesonder KCl als günstig erwiesen, wobei die Konzentration zwischen 2 bis 200 mmol/l, vorzugsweise zwischen 5 bis 150 mmol/l, insbesondere zwischen 10 bis 100 mmol/l liegt.

Die Glutamat-Dehydrogenase-Aktivität läßt sich durch Zusatz von ADP steigern; für die ADP-Konzentration haben sich Werte zwischen 0,05 und 2 mmol/l, vorzugsweise zwischen 0,1 und 1,5 mmol/l, insbesondere zwischen 0,3 und 1 mmol/l als günstig erwiesen.

Neben den genannten Bestandteilen kann das Reagenz noch Hilfsstoffe wie Konservierungsmittel, z.B. Na-Azid, Detergentien und Lipasen zur Aufklarung von durch Triglyceriden getrübten Proben enthalten.

Die Reagenzbestandteile können außerdem aus Gründen des Meßverfahrens wie auch der Stabilität z.T. getrennt voneinander in zwei oder mehreren wäßrigen gepufferten Lösungen vorliegen; insbesondere ist es zweckmäßig, die Creatinin-Iminohydrolase (im Hinblick auf eine evtl. notwendige Testentstörung bezüglich endogenen Proben-$NH_4^+$-Gehalts) zusammen mit ATP (wegen einer möglichen unspezifischen-ATPase-Nebenaktivität der NMHase) von den übrigen Komponenten zu separieren. Aktivitäten bzw. Konzentrationen sind dabei so einzustellen, daß nach Vermischung der getrennten Lösungen zum kompletten Reagenz die o.g. Werte wieder erreicht werden.

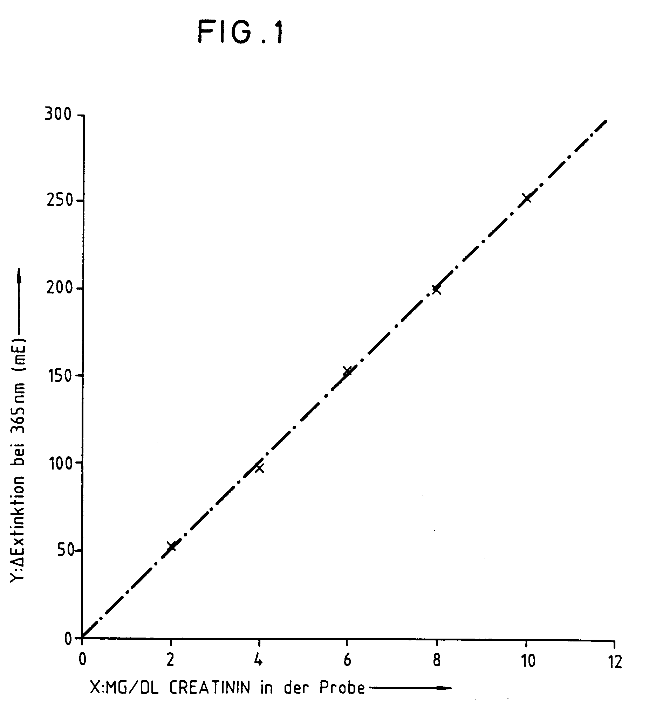

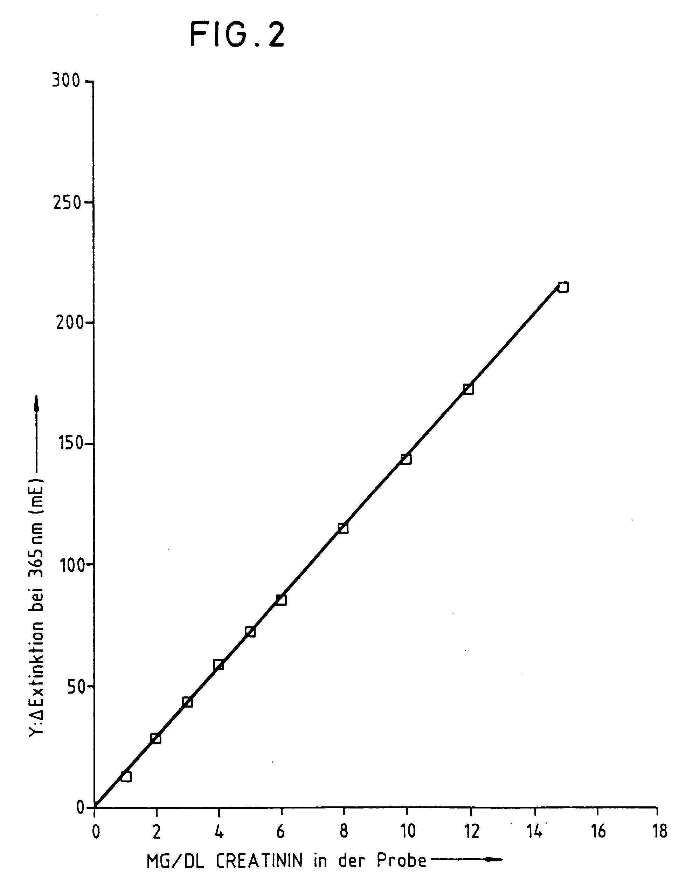

Die folgenden Beispiele erläutern die Erfindung zusammen mit den Abbildungen 1 und 2 weiter.

Es zeigen:

Abb. 1       die Creatinin-Bestimmung nach dem Probe-/Probenleerwertverfahren,

Abb. 2       die Creatinin-Bestimmung nach dem Stufenmeßverfahren.

Folgende Abkürzungen und Synonyma werden verwendet:

CRIMI:       Creatinin-Iminohydrolase

NMHase:       ATP-abhängige 1-Methylhydantoinase

CSHase:       N-Carbamoylsarcosin-Amidohydrolase

GIDH:       Glutamat-Dehydrogenase

$\alpha$-KG:       $\alpha$-Ketoglutarat-Na$_2$

TRIS:       Tris(hydroxymethyl)aminomethan

TES:       2-{[Tris-(hydroxymethyl)methyl-]}amino-ethansulfonsäure

**Beispiel 1**

Enzymatische Creatinin-Bestimmung: Manuelle Durchführung nach der Endpunktmethode (Probe-/Probenleerwertverfahren)

1.1 Reagenzien:

| Reagenz 1: | |
| --- | --- |
| Komponente | Konzentration im Reagenz |
| TRIS-HCl (pH 8,2) | 100 mmol/l |
| KCl | 100 mmol/l |
| $\alpha$-KG | 10 mmol/l |
| MgCl$_2$ | 5 mmol/l |
| NADH | 250 $\mu$mol/l |
| ADP | 500 $\mu$mol/l |
| CSHase | 5 U/ml |
| NMHase | 0,5 U/ml |
| GIDH | 60 U/ml |

| Reagenz 2: | |
| --- | --- |
| Komponente | Konzentration im Reagenz |
| TRIS-HCl (pH 8,2) | 100 mmol/l |
| ATP | 90 mmol/l |
| CRIMI | 20 U/ml |

1.2 Probenmaterial: wäßrige Creatinin-Lösungen mit 2, 4, 6, 8 und 10 mg/dl.

1.3 Testdurchführung:

T = 25 °C; Wellenlänge 365 nm; Schichtdicke = 10 mm Messung gegen Luft.

In Küvetten pipettieren:

|  | Probe | Probenleerwert (PLW) |
|---|---|---|
| Reagenz 1 | 2,00 ml | 2,00 ml |
| Reagenz 2 | 0,40 ml | - |
| dest. $H_2O$ | - | 0,40 ml |

mischen, $E_1$ Probe und $E_1$ PLW messen, dann zugeben:

| Probe | 0,10 ml | 0,10 ml |
|---|---|---|

erneut mischen, 15 min inkubieren, dann $E_2$ Probe und $E_2$ PLW messen.

$E = (E_1 \text{ Probe} - E_2 \text{ Probe}) - (E_1 \text{ PLW} - E_2 \text{ PLW})$.

Die Abhängigkeit der gemessenen Extinktionswerte (E) von der Creatinin-Konzentration in der Probe ist in Abb. 1 dargestellt.

Dieses Beispiel belegt, daß mit dem erfindungsgemäßen Verfahren tatsächlich die doppelte Nachweisempfindlichkeit gegenüber den bekannten enzymatischen Creatinin-UV-Tests erreicht wird:

Während gemäß Beispiel 1 bei Einsatz von 100 µl Probe in 2,5 ml Gesamt-Testgemisch pro mg Creatinin/dl Probe experimentell ein Extinktionswert von durchschnittlich 25,4 mE (Steigung der Regressionsgeraden) erzielt wird (Theorie: 24,7 mE), berechnet sich rein theoretisch unter gleichem Probe-/Gesamt-Testgemisch-Volumenverhältnis für die bisher bekannten Creatinin-UV-Tests aus dem molaren Extinktionskoeffizienten von NAD(P)H bei 365 nm ein Wert von nur 12,3 mE.

**Beispiel 2**

Enzymatische Creatinin-Bestimmung: Automatisierte Durchführung an einem Hitachi 704 Spektrometer nach der kinetischen 'fixed-time'-Methode ($E_1$-/$E_2$-Verfahren)

2.1 Reagenzien:

| Reagenz 1: | |
|---|---|
| Komponente | Konzentration im Reagenz |
| TRIS-HCl (pH 8,2) | 100 mmol/l |
| KCl | 15 mmol/l |
| $\alpha$-KG | 15 mmol/l |
| NADH | 350 µmol/l |
| CSHase | 8 U/ml |
| NMHase | 1 U/ml |
| GlDH | 90 U/ml |

| Reagenz 2: | |
|---|---|
| Komponente | Konzentration im Reagenz |
| TRIS-HCl (pH 8,2) | 100 mmol/l |
| MgCl$_2$ | 30 mmol/l |
| ATP | 30 mmol/l |
| CRIMI | 15 U/ml |

2.2 Probenmaterial: Wäßrige Creatinin-Lösungen mit 1, 2, 3, 4, 5, 6, 8, 10, 12 und 15 mg/dl.

2.3 Testdurchführung:

T = 37°C; Wellenlängen (bichromatische Messung) 340/415 nm; Schichtdicke 7 mm.

14 $\mu$l Probe mit 350 $\mu$l Reagenz 1 vermischen, 5 min inkubieren, dann 50 $\mu$l Reagenz 2 zupipettieren und die Extinktionsänderung zwischen dem 19. und 32. Meßzyklus (d.h. zwischen sec. 51.4 und sec. 304 nach Zugabe von Reagenz 2) messen.

Die Abhängigkeit der Extinktionsänderung von der Creatinin-Konzentration in der Probe ist in Abb. 2 dargestellt.

Damit ist gezeigt, daß das erfindungsgemäße Verfahren zur enzymatischen Creatinin-Bestimmung auch prinzipiell gut für die Applikation an automatisierten Analysengeräten geeignet ist.

**Patentansprüche**

1.  Verfahren zur enzymatischen Bestimmung von Creatinin in wäßrigen Lösungen, wobei man das in der Probe enthaltene Creatinin mittels der Creatinin-Iminohydrolase zu 1-Methylhydantoin und einem ersten Molekül NH$_3$ umsetzt,
    **dadurch gekennzeichnet,**
    daß man das gebildete 1-Methylhydantoin mittels der ATP-abhängigen 1-Methylhydantoinase und der N-Carbamoylsarcosin-Amidohydrolase zu Sarcosin, CO$_2$ und einem zweiten Molekül NH$_3$ umsetzt und beide gebildeten Moleküle NH$_3$ gemeinsam bestimmt.

2.  Verfahren nach Anspruch 1,
    **dadurch gekennzeichnet,**
    daß man die beiden gebildeten Moleküle NH$_3$ mit $\alpha$-Ketoglutarat in Gegenwart von Glutamat-Dehydrogenase sowie NADH oder NADPH zu Glutamat umsetzt und den Verbrauch an NADH bzw. NADPH mißt.

3.  Verfahren nach Anspruch 1 oder 2,
    **dadurch gekennzeichnet,**
    daß Lösungen von Creatinin-Iminohydrolase und ATP sowie gegebenenfalls CSHase (N-Carbamoylsarcosin-Amidohydrolase) getrennt von den anderen Komponenten zubereitet werden und erst kurz vor Reaktionsbeginn mit den anderen Komponenten vermischt werden.

4.  Verfahren nach einem der Ansprüche 1 bis 3,
    **dadurch gekennzeichnet,**
    daß man in einer ersten Probe (Leerwert) den endogenen NH$_3$-Gehalt der Lösung in Abwesenheit von Creatinin-Iminohydrolase bestimmt und endogenes NH$_3$ in einer getrennten Probe bestimmt und bei der Berechnung der Creatininmenge berücksichtigt.

5.  Reagenz zur enzymatischen Bestimmung von Creatinin in wäßrigen Lösungen,
    **dadurch gekennzeichnet,**
    daß es Creatinin-Iminohydrolase, ATP-abhängige 1-Methylhydantoinase, N-Carbamoylsarcosin-Amidohydrolase, ATP, Mg$^{++}$ und eine Puffersubstanz enthält.

6.  Reagenz nach Anspruch 5,
    **dadurch gekennzeichnet,**

daß es 0,2 bis 20 U/ml Creatinin-Iminohydrolase, 0,1 bis 10 U/ml 1-Methylhydantoinase, 0,5 bis 20 U/ml N-Carbamoylsarcosin-Amidohydrolase, 0,05 bis 50 mmol/l ATP, 0,1 bis 50 mmol $Mg^{2+}$-Ionen und 20 bis 500 mmol/l einer Puffersubstanz enthält, wobei der pH-Wert des Reagenz zwischen 7,0 und 9,0 liegt.

7.  Reagenz nach Anspruch 6,
    **dadurch gekennzeichnet,**
    daß es 1 bis 10 U/ml Creatinin-Iminohydrolase, 0,4 bis 2 U/ml 1-Methylhydantoinase, 2 bis 10 U/ml N-Carbamoylsarcosin-Amidohydrolase, 1 bis 15 mmol/l ATP, 1 bis 10 mmol/l $Mg^{2+}$-Ionen und 75 bis 150 mmol/l einer Puffersubstanz enthält, wobei der pH-Wert des Reagenz zwischen 7,7 und 8,4 liegt.

8.  Reagenz nach einem der Ansprüche 5 bis 7,
    **dadurch gekennzeichnet,**
    daß es zusätzlich 2 bis 200 mmol/l, vorzugsweise 10 bis 100 mmol/l $K^+$-Ionen enthält.

9.  Reagenz nach einem der Ansprüche 5 bis 8,
    **dadurch gekennzeichnet,**
    daß es zusätzlich Glutamat-Dehydrogenase, $\alpha$-Ketoglutarat und NADH oder NADPH enthält.

10. Reagenz nach Anspruch 9,
    **dadurch gekennzeichnet,**
    daß es zusätzlich 0,05 bis 2 mmol/l, vorzugsweise 0,3 bis 1 mmol/l ADP enthält.

**Claims**

1.  Method for the enzymatic determination of creatinine in agueous solutions, in which the creatinine contained in the sample is converted to 1-methylhydantoin and a first molecule of $NH_3$ by creatinine iminohydrolase,
    **wherein**
    the 1-methylhydantoin formed is converted to sarcosine, $CO_2$ and a second molecule of $NH_3$ by the ATP-dependent 1-methylhydantoinase and N-carbamoylsarcosine amidohydrolase and both $NH_3$ molecules which form are determined together.

2.  Method as claimed in claim 1,
    **wherein**
    both the $NH_3$ molecules which form are converted with $\alpha$-ketoglutarate to glutamate in the presence of glutamate dehydrogenase and NADH or NADPH and the consumption of NADH or NADPH is measured.

3.  Method as claimed in claims 1 or 2,
    **wherein**
    solutions of creatinine iminohydrolase and ATP as well as, if desired, CSHase (N-carbamoylsarcosine amidohydrolase) are prepared separately from the other constituents and are only mixed with the other constituents shortly before the reaction is started.

4.  Method as claimed in one of the claims 1 to 3,
    **wherein**
    the endogenous $NH_3$ content of the solution is determined in a first sample (blank value) in the absence of creatinine iminohydrolase and endogenous $NH_3$ is determined in a separate sample and taken into consideration in the calculation of the amount of creatinine.

5.  Reagent for the enzymatic determination of creatinine in aqueous solutions,
    **wherein**
    it contains creatinine iminohydrolase, ATP-dependent 1-methylhydantoinase, N-carbamoylsarcosine amidohydrolase, ATP, $Mg^{++}$ and a buffer substance.

6.  Reagent as claimed in claim 5,
    **wherein**
    it contains 0.2 to 20 U/ml creatinine iminohydrolase, 0.1 to 10 U/ml 1-methylhydantoinase, 0.5 to 20 U/ml N-carbamoylsarcosine amidohydrolase, 0.05 to 50 mmol/l ATP, 0.1 to 50 mmol $Mg^{2+}$ ions and 20 to 500 mmol/l of a buffer substance, and the pH value of the reagent is between 7.0 and 9.0.

**7.** Reagent as claimed in claim 6,

**wherein**

it contains 1 to 10 U/ml creatinine iminohydrolase, 0.4 to 2 U/ml 1-methylhydantoinase, 2 to 10 U/ml N-carbamoylsarcosine amidohydrolase, 1 to 15 mmol/l ATP, 1 to 10 mmol/l $Mg^{2+}$ ions and 75 to 150 mmol/l of a buffer substance, and the pH value of the reagent is between 7.7 and 8.4.

**8.** Reagent as claimed in one of the claims 5 to 7,

**wherein**

it additionally contains 2 to 200 mmol/l, preferably 10 to 100 mmol/l $K^+$ ions.

**9.** Reagent as claimed in one of the claims 5 to 8,

**wherein**

it additionally contains glutamate dehydrogenase, $\alpha$-ketoglutarate and NADH or NADPH.

**10.** Reagent as claimed in claim 9,

**wherein**

it additionally contains 0.05 to 2 mmol/l, preferably 0.3 to 1 mmol/l ADP.

**Revendications**

**1.** Procédé de détermination enzymatique de la créatinine dans des solutions aqueuses, dans lequel, à l'aide de la créatinine-iminohydrolase, on convertit la créatinine contenue dans l'échantillon en 1-méthylhydantoïne et en une première molécule de $NHPts_3$, caractérisé en ce que, à l'aide de la 1-méthylhydantoïnase dépendante de l'ATP et de la N-carbamoylsarcosine-amidohydrolase, on convertit la 1-méthylhydantoïne formée en sarcosine, en $CO_2$ et en une seconde molécule de $NH_3$ et on détermine ensemble les deux molécules de $NH_3$ formées.

**2.** Procédé selon la revendication 1, caractérisé en ce que l'on convertit en glutamate les deux molécules de $NH_3$ formées avec l'$\alpha$-cétoglutarate en présence de glutamate-déshydrogénase et de NADH ou NADPH et on mesure la consommation de NADH ou de NADPH.

**3.** Procédé selon la revendication 1 ou 2, caractérisé en ce que des solutions de créatinine-iminohydrolase et d'ATP et éventuellement de CSHase (N-carbamoylsarcosine-amidohydrolase) sont préparées séparément des autres constituants et ne sont mélangées avec les autres constituants que peu de temps avant le début de la réaction.

**4.** Procédé selon l'une des revendications 1 à 3, caractérisé en ce que l'on détermine dans un premier échantillon (valeur témoin) la teneur en $NHPts_3$ endogène de la solution en l'absence de créatinine-iminohydrolase et on détermine le $NH_3$ endogène dans un échantillon séparé et on en tient compte dans le calcul de la quantité de créatinine.

**5.** Réactif de détermination enzymatique de la créatinine dans des solutions aqueuses, caractérisé en ce qu'il contient de la créatinine-iminohydrolase, de la 1-méthylhydantoïnase dépendante de l'ATP, de la N-carbamoylsarcosine-amidohydrolase, de l'ATP, $Mg^{++}$ et une substance tampon.

**6.** Réactif selon la revendication 5, caractérisé en ce qu'il contient 0,2 à 20 U/ml de créatinine-iminohydrolase, 0,1 à 10 U/ml de 1-méthylhydantoïnase, 0,5 à 20 U/ml de N-carbamoylsarcosine-amidohydrolase, 0,05 à 50 mmol/l d'ATP, 0,1 à 50 mmol/l d'ions $Mg^{2+}$ et 20 à 500 mmol/l d'une substance tampon, le pH du réactif étant situé entre 7,0 et 9,0.

**7.** Réactif selon la revendication 6, caractérisé en ce qu'il contient 1 à 10 U/ml de créatinine-iminohydrolase, 0,4 à 2 U/ml de 1-méthylhydantoïnase, 2 à 10 U/ml de N-carbamoylsarcosine-amidohydrolase, 1 à 15 mmol/l d'ATP, 1 à 10 mmol/l d'ions $Mg^{2+}$ et 75 à 150 mmol/l d'une substance tampon, le pH du réactif étant situé entre 7,7 et 8,4.

8. Réactif selon l'une des revendications 5 à 7, caractérisé en ce qu'il contient en outre 2 à 200 mmol/l, de préférence 10 à 100 mmol/l d'ions K$^+$.

9. Réactif selon l'une des revendications 5 à 8, caractérisé en ce qu'il contient en outre de la glutamate-déshydrogénase, de l'α-cétoglutarate et NADH ou NADPH.

10. Réactif selon la revendication 9, caractérisé en ce qu'il contient en outre 0,05 à 2 mmol/l, de préférence 0,3 à 1 mmol/l d'ADP.

# FIG.1

# FIG. 2